(19) Europäisches Patentamt
European Patent Office
Office européen des brevets

(11) EP 0 939 638 B1

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(21) Anmeldenummer: **97944849.5**

(22) Anmeldetag: **08.09.1997**

(51) Int Cl.[7]: **A61K 31/70**, A61K 47/10, A61K 47/14, A61K 47/44

(86) Internationale Anmeldenummer:
**PCT/EP97/04867**

(87) Internationale Veröffentlichungsnummer:
**WO 98/011902 (26.03.1998 Gazette 1998/12)**

(54) **INJEKTIONSFORMULIERUNGEN VON AVERMECTINEN UND MILBEMYCINEN**
INJECTABLE FORMULATIONS OF AVERMECTINS AND MILBEMYCINS
FORMULATIONS INJECTABLES D'AVERMECTINES ET DE MILBEMYCINES

(84) Benannte Vertragsstaaten:
**AT BE CH DE DK ES FR GB GR IE IT LI NL**

(30) Priorität: **18.09.1996 DE 19638045**

(43) Veröffentlichungstag der Anmeldung:
**08.09.1999 Patentblatt 1999/36**

(73) Patentinhaber: **BAYER AG**
**51368 Leverkusen (DE)**

(72) Erfinder:
- **GROSSE-BLEY, Michael**
  **D-51061 Köln (DE)**
- **KUJANEK, Richard**
  **D-51061 Köln (DE)**

(56) Entgegenhaltungen:
**EP-A- 0 045 655**
**EP-A- 0 146 414**
**EP-A- 0 393 890**
**EP-A- 0 413 538**
**EP-A- 0 525 307**
**EP-A- 0 535 734**
**WO-A-97/11709**
**WO-A-97/37653**
**GB-A- 2 275 193**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Injektionsformulierungen von Avermectinen und Milbemycinen auf Basis von Lösungsmittelmischungen, die Sesamöl enthalten.

Injektionsformulierungen von Ivermectin sind bekannt aus EP-A 146 414. Die Formulierungen enthalten ein Lösemittelgemisch aus Propylenglykol und Glycerinformal im Verhältnis 60:40 V/V. Von Propylenglykol ist bekannt, daß es in bestimmten Konzentrationen lokale Unverträglichkeiten hervorrufen kann (siehe Review: B. Kruss, Acta Pharm. Technol. 35(4) (1989) 187-196). Auch kann es zur Ausfällung des wasserunlöslichen Wirkstoffs Ivermectin im Gewebe um die Applikationsstelle kommen. So wurden bei der Anwendung entsprechender Formulierungen deutliche Schwellungen und Gewebeunverträglichkeiten an den Injektions-. stellen beobachtet, die sich zum Teil erst nach mehreren Wochen zurückbildeten.

Injektionsformulierungen bestimmter Avermectine sind bekannt aus EP-A 393 890. Es handelt sich um ölige Formulierungen auf Basis von Sesamöl und Ethyloleat im Verhältnis 90:10 V/V. Diese Formulierungen sind verträglich, haben aber den Nachteil, daß die Löslichkeit für Avermectine/Milbemycine oft nicht ausreicht, um eine für die Anwendung gewünschte Konzentration von 1 % M/V oder höher zu erreichen. In der Regel erhält man bei erhöhten Temperaturbedingungen ($T \geq 80°C$) übersättigte 1 % M/V-Lösungen, die bei tieferen Temperaturen auf Dauer wieder auskristallisieren.

Weitere Injektionsformulierungen von Avermectinen sind bekannt aus EP-A 45 655. Die dort beschriebenen Formulierungen enthalten verhältnismäßig hohe Anteile an Emulgatoren und sind zum Teil wenig verträglich.

Injektionsformulierungen von Avermectinen, die Triacetin (Glycerintriacetat) enthalten, sind in EP-A 413 538 beschrieben. In EP-A 535 734 werden Injektionsformulierungen von Avermectinen auf Basis von Triacetin und hydriertem Rizinusöl beschrieben.

Weitere Formulierungen zur Injektion von Milbemycinen und Avermectinen sind in EP-A 525 307 beschrieben. Die Herstellung der Formulierungen erfolgt, indem Glycerintristearat mit dem Wirkstoff geschmolzen und mit einem öligen neutralen Triglycerid vermischt und unter Verwendung von z.B. Methylcellulose und Salzen emulgiert wird. Die durchschnittliche Partikelgröße in der so erhaltenen Mikroemulsion soll zwischen 25 und 300 µm liegen.

Gegenstand der vorliegenden Erfindung sind Injektionsformulierungen von Avermectinen und Milbemycinen auf Basis eines Lösungsmittelgemischs, enthaltend Sesamöl, mittelkettige Triglyceride oder Glycolester oder Fettsäureester und einem weiteren Solvenz.

Die Formulierungen enthalten bevorzugt

1. Wirkstoff 0,2 bis 5 % M/V;

2. Sesamöl 60 bis 90 % V/V;

3. mittelkettige Triglyceride oder Glycolester oder Fettsäureester 10 bis 30 Vol.-%;

4. 1 bis 20 Vol.-% Benzylalkohol oder Propylenglykol oder andere geeignete aliphatische oder aromatische ein- oder mehrwertige Alkohole und deren Derivate (z.B. cyclische Carbonate, Acetate, Acetale/Ketale) oder Rizinusöl;

5. gegebenenfalls weitere Hilfsstoffe.

Die erfindungsgemäßen Formulierungen weisen eine hervorragende Löslichkeit für die Wirkstoffe auf.

Die hohe Viskosität von Sesamöl kann durch Zusatz von mittelkettigen Triglyceriden oder Propylenglykoloctanoat/decanoat oder besonders Ethyloleat auf ein gewünschtes niedriges Maß eingestellt werden. Zusätzlich kann durch Addition von kleineren Volumina hydrophiler Lösemittel wie Benzylalkohol, Propylenglykol oder Propylencarbonat unter Beibehaltung eines einphasigen Systems die Löslichkeit des Wirkstoffs verbessert, die Viskosität weiter herabgesetzt und die Bioverfügbarkeit des Wirkstoffs verbessert werden. Als einziges Triglycerid weist Rizinusöl ein hohes Lösepotential für die in Frage stehenden Wirkstoffe auf.

Die in den erfindungsgemäßen Formulierungen eingesetzten Wirkstoffe sind bekannt.

Avermectine wurden aus dem Mikroorganismus Streptomyces avermitilis als mikrobielle Metabolite isoliert (US-Pat. 4 310 519) und können im wesentlichen als Gemisch, bestehend aus den acht Komponenten $A_{1a}$, $A_{1b}$, $A_{2a}$, $A_{2b}$, $B_{1a}$, $B_{1b}$, $B_{2a}$ und $B_{2b}$, auftreten (I. Putter et al. Experentia 37 (1981) S. 963, Birkhäuser Verlag (Schweiz)). Daneben besitzen auch die synthetischen Derivate, insbesondere das 22,23 Dihydroavermectin $B_1$ (Ivermectin), Interesse (US-Pat. 4 199 569). Milbemycin B-41 D wurde fermentativ aus Streptomyces hygroscopicus isoliert (vgl. "Milbemycin: Discovery and Development" I. Junya et al. Annu. Rep. Sankyo Res. Lab. 45 (1993), S. 1-98; JP-Pat. 8 378 549; GB 1 390 336).

Die Verwendung der Avermectine, z.B. 22,23-Dihydroavermectine $B_1$ (Ivermectin), und Milbemycine als Endoparasitizide ist bekannt und Gegenstand zahlreicher Patentanmeldungen sowie Übersichtsartikel (z.B. Biologische Wirkungen in: "Ivermectin and Abamectin" W.C. Campbell, Ed., Springer Verlag, New York, N.Y., 1989; "Avermectins and Milbemycins Part II" H.G. Davies et al. Chem. Soc. Rev. 20 (1991) S. 271-339; Chemische Modifikationen in: G. Lukacs et al. (Eds.), Springer Verlag, New York, (1990), Chapter 3; Cydectin® [Moxidectin und Derivate]: G.T. Carter et al. J. Chem. Soc. Chem. Com-mun. (1987), S. 402-404); EP 423 445-A1) "Doramectin - a potent novel endectozide" A.C. Goudie et al. Vet. Parasitol. 49 (1993), S. 5-15).

Besonders hervorgehoben seien Avermectine und deren Derivate der allgemeinen Formel (I)

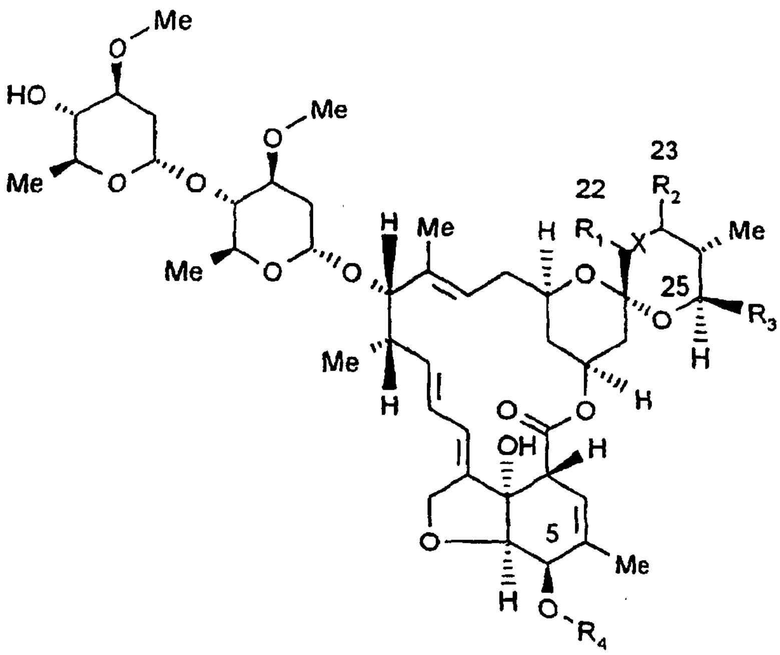

(I),

in welcher
die Reste $R^1$ bis $R^4$ die in der nachfolgenden Tabelle 1 angegebene Bedeutung haben und X für eine Einfach- oder Doppelbindung zwischen der $C_{22}$- und $C_{23}$-Position ($-C_{22}R^1-X-C_{23}R^2-$) stehen kann.

Im Falle einer Doppelbindung befinden sich keine Substituenten ($R^1$, $R^2$) an der $C_{22}$- und $C_{23}$-Position.

Tabelle 1

| **Makrocyclisches Lacton** | **$-C_{22}R^1-X-C_{23}R^2-$** | **$R^3$** | **$R^4$** |
|---|---|---|---|
| Avermectin $A_{1a}$ | -CH=CH- | -sec-Bu | -Me |
| Avermectin $A_{1b}$ | -CH=CH- | -iso-Pr | -Me |
| Avermectin $A_{2a}$ | $-CH_2$-CHOH- | -sec-Bu | -Me |
| Avermectin $A_{2b}$ | $-CH_2$-CHOH- | -iso-Pr | -Me |
| Avermectin $B_{1a}$ | -CH=CH- | -sec-Bu | -H |
| Avermectin $B_{1b}$ | -CH=CH- | -iso-Pr | -H |
| Avermectin $B_{2a}$ | $-CH_2$-CHOH- | -sec-Bu | -H |
| Avermectin $B_{2b}$ | $-CH_2$-CHOH- | -iso-Pr | -H |
| 22,23-Dihydroavermectin $B_{1a}$ | $-CH_2-CH_2-$ | -sec-Bu | -H |
| 22,23-Dihydroavermectin $B_{1b}$ | $-CH_2-CH_2-$ | -iso-Pr | -H |
| Doramectin | -CH=CH- | -Chx | -H |
| 22,23-Dihydroavermectin $B_1$ steht für Ivermectin; sec-Bu = sekundär Butyl; iso-Pr = Isopropyl; Chx = Cyclohexyl; -Me = Methyl | | | |

Die Avermectine und 22,23-Dihydroavermectine $B_1$ (Ivermectin) der allgemeinen Formel (I) werden in der Regel als Gemische eingesetzt. Von besonderem Interesse ist hierbei das Produkt Abamectin, das die Avermectine $B_1$ enthält, und deren Hydrierungsprodukte, die 22,23-Dihydroavermectine $B_1$ (Ivermectin).

Die mit "b" bezeichneten Verbindungen der makrocyclischen Lactone, die in der $C_{25}$-Position einen iso-Propylrest besitzen, müssen nicht notwendigerweise von den "a" Verbindungen, welche eine sec-Butylgruppe in der $C_{25}$-Position haben, getrennt werden. Es wird generell das Gemisch beider Substanzen, bestehend aus > 80 % sec-Butylderivat ($B_{1a}$) und < 20 % iso-Propylderivat ($B_{1b}$) isoliert, und kann erfindungsgemäß verwendet werden. Zudem können bei den Stereoisomeren die Substituenten in der $C_{13}$- und $C_{23}$-Position sowohl α- als auch β-ständig am Ringsystem angeordnet sein, d. h. sich oberhalb oder unterhalb der Molekülebene befinden. In jedem Fall werden alle Stereoisomeren erfindungsgemäß berücksichtigt.

Besonders genannt seien die Milbemycine. Die Milbemycine haben die gleiche makrolide Ringstruktur wie die Avermectine oder 22,23-Dihydroavermectine $B_1$ (Ivermectine), tragen aber keinen Substituenten (d.h. fehlendes Oleandrose-Disaccharidfragment) in Position 13 ($R^5$ = Wasserstoff).

Beispielhaft seien als Milbemycine aus der Klasse der macrocyclischen Lactone die Verbindungen mit der allgemeinen Formel (II) genannt

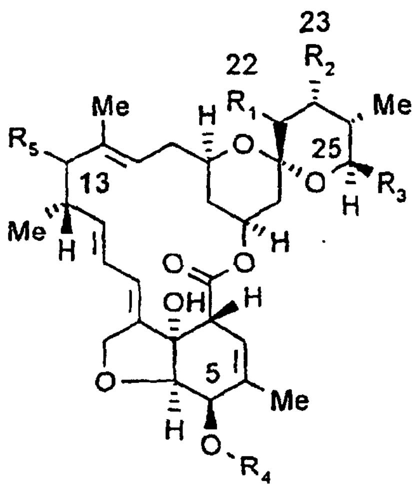

(II)

in welcher
die Reste $R^1$ bis $R^5$ die in der nachfolgenden Tabelle 2 angegebene Bedeutung haben:

Tabelle 2

| Makrocyclisches Lacton | $R^1$ | $R^2$ | $R^3$ | $R^4$ | $R^5$ |
|---|---|---|---|---|---|
| Milbemycin B41 D | -H | -H | -iso-Pr | -H | -H |
| Nemadectin | -H | -OH | 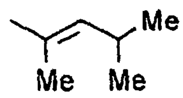 | -H | -H |
| Moxidectin | -H | =N-O-Me | 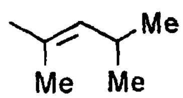 | -H | -H |

iso-Pr = Isopropyl

Ganz besonders hervorgehoben seien die Wirkstoffe

Avermectin $B_{1a}/B_{1b}$ (Abamectin),
22,23-Dihydroavermectin $B_{1a}/B_{1b}$ (Ivermectin),
Doramectin,
Moxidectin.

Die Wirkstoffe liegen in den erfindungsgemäßen Formulierungen in Konzentrationen von 0,2 bis 5 %, bevorzugt von 0,5 bis 2 %, besonders bevorzugt 1 % M/V vor.

Das in den erfindungsgemäßen Formulierungen eingesetzte Sesamöl (60 bis 90 % V/V) ist bekannt.

Die in den erfindungsgemäßen Formulierungen eingesetzten Viskositätserniedriger, insbesondere Ethyloleat, sind bekannt.

Gute und als Bestandteil von Injektabilia einsetzbare weitere Lösungsmittel für die Wirkstoffe sind namentlich Benzylalkohol, Propylenglykol, Glycerinformal, Propylencarbonat, Triacetin, die Myvacete® (Warenzeichen von Eastman), Propylenglykoldiacetat, Polyethylenglykol 400, Tetraglykol sowie Rizinusöl. Besonders bevorzugt sind Benzylalkohol (1 bis 5 % V/V) und Rizinusöl (10 bis 20 % V/V).

Die Löslichkeit von Ivermectin beträgt in Benzylalkohol > 40 Gew.-%, in Rizinusöl ~ 4 Gew.-%.

Weitere Zusätze zu den erfindungsgemäßen Formulierungen sind Stabilisatoren wie Butylhydroxyanisol (BHA), Butylhydroxytoluol (BHT) oder Propylgallat von insgesamt bis zu 1000 ppm. Besonders geeignete Stabilisatorkombinationen und -konzentrationen sind z.B. 100 ppm BHA oder 100 ppm BHA plus 150 ppm Propylgallat oder 200 ppm BHA plus 100 ppm Propylgallat.

Die Viskosität der erfindungsgemäßen Formulierungen liegt zwischen 20 bis 60 mPa.s (20°C), bevorzugt zwischen 25 bis 55 mPa.s (20°C), besonders bevorzugt zwischen 30 und 51 mPa.s (20°C).

Die folgenden Beispiele erläutern die Erfindung.

Anmerkung:

$$V/V = \frac{\text{Volumen}}{\text{Volumen}} \text{ entspricht Volumenprozent}$$

$$M/V = \frac{Masse}{Volumen}$$

1 % M/V heißt z.B. 10 mg Wirkstoff in 1 ml Lösung.

**Beispiel 1**

| | |
|---|---|
| Sesamöl | q.s. 100 % V/V |
| Ethyloleat | 10 % V/V |
| Benzylalkohol | 2 % V/V |
| Ivermectin | 1 % M/V |
| Butylhydroxyanisol (BHA) | 100 ppm (≙ 0,01 % M/V) |
| Dichte: | 0,922 g/ml |
| Viskosität: | 44 mPa.s bei 20°C |
| | 85 mPa.s bei 5°C |
| | 24 mPa.s bei 39°C |

**Beispiel 2**

| | |
|---|---|
| Sesamöl | q.s. 100 % V/V |
| Ethyloleat | 20 % V/V |
| Rizinusöl | 10 % V/V |
| Ivermectin | 1 % M/V |
| Butylhydroxyanisol (BHA) | 100 ppm (≙ 0,01 % M/V) |
| Dichte: | 0,927 g/ml |
| Viskosität: | 38 mPa.s bei 20°C |
| | 83 mPa.s bei 5°C |

Allgemeine Herstellvorschrift für die Beispiele 1 und 2 als sterile Lösungen zur Injektion:

Sesamöl und Ethyloleat, mit 100 ppm BHA versehen, werden in einen Edelstahlbehälter eingewogen und unter Rühren homogenisiert. Unter weiterem Rühren wird das Ivermectin, in Benzylalkohol oder Rizinusöl gelöst bzw. angelöst, eingebracht. Die Mischung wird auf 40 bis 60°C erwärmt, um die rasche, vollständige Auflösung des Wirkstoffs zu garantieren (alles unter Stickstoffbegasung).

Dann wird bei gleicher Temperatur über ein 0,22 µm Filter sterilfiltriert (in der Regel wird ein 0,45 µm oder 1 µm Filter vorgeschaltet). Es folgt aseptische Abfüllung in Braunglasflaschen.

Die so hergestellten Formulierungen sind bei der Anwendung am Rind hervorragend verträglich. Sie sind außerdem über mindestens 6 Wochen bei Temperaturen von 60°C lagerstabil.

**Patentansprüche**

**1.** Injektionsformulierungen der folgenden Zusammensetzung:
0,2 bis 5 % M/V eines Avermectins oder Milbemycins in einem Lösungsmittelgemisch bestehend aus

- 60 bis 90 % V/V Sesamöl, sowie

- 10 bis 30 % V/V Ethyloleat oder Capryl-Caprinsäure-Triglycerid (Miglyol®812) oder Propylenglykoldiester der Capryl-Caprinsäure (Miglyol®840) und
- 1 bis 5 % V/V Benzylalkohol oder 10 bis 20 % V/V Rizinusöl

sowie gegebenenfalls bis zu 1000 ppm Stabilisatoren.

**2.** Formulierungen gemäß Anspruch 1 der folgenden Zusammensetzung: 1 % M/V Ivermectin, 65 bis 90 % V/V Sesamöl, 10 bis 20 % V/V Ethyloleat und 1 bis 3 % V/V Benzylalkohol oder 10 % V/V Rizinusöl sowie gegebenenfalls bis zu 500 ppm Stabilisatoren.

**3.** Verfahren zur Herstellung der Formulierungen gemäß Anspruch 1, **dadurch gekennzeichnet, dass** man den Wirkstoff in Rizinusöl oder Benzylalkohol (an)löst und die weiteren in Anspruch 1 angegebenen Komponenten zufügt oder, dass man den Wirkstoff in einer Mischung aus den in Anspruch 1 angegebenen Komponenten auflöst.

**4.** Verwendung von Rizinusöl oder Benzylalkohol als Lösungsverbesserer in einer Formulierung gemäß Anspruch 1.

## Claims

**1.** Injection formulations of the following composition:
0.2 to 5% m/v of an avermectin or milbemycin in a solvent mixture consisting of

- 60 to 90% v/v of sesame oil, and
- 10 to 30% v/v of ethyl oleate or caprylic/capric triglyceride (Miglyol®812) or propylene glycol dicaprylate/dicaprate (Miglyol®840) and
- 1 to 5% v/v of benzyl alcohol or 10 to 20% v/v of castor oil

and optionally up to 1000 ppm of stabilizers.

**2.** Formulations according to Claim 1 of the following composition:
1% m/v of ivermectin, 65 to 90% v/v of sesame oil, 10 to 20% v/v of ethyl oleate and 1 to 3% v/v of benzyl alcohol or 10% v/v of castor oil and optionally up to 500 ppm of stabilizers.

**3.** Process for the preparation of the formulations according to Claim 1,
**characterized in that** the active compound is (partially) dissolved in castor oil or benzyl alcohol and the other components specified in Claim 1 are added or **in that** the active compound is dissolved in a mixture of the components specified in Claim 1.

**4.** Use of castor oil or benzyl alcohol as solubilizers in a formulation according to Claim 1.

## Revendications

**1.** Formulations injectables possédant la composition indiquée ci-après :
de 0,2 à 5 % M/V d'une avermectine ou d'une milbemycine dans un mélange de solvants constitué par

- de l'huile de sésame à raison de 60 à 90 % V/V, et
- de l'oléate d'éthyle ou du triglycéride d'acide caprylique - caprique (Miglyol® 812) ou encore du diester de propylèneglycol de l'acide caprylique - caprique (Miglyol® 840) à raison de 10 à 30 % V/V et
- de l'alcool benzylique à raison de 1 à 5 % ou de l'huile de ricin à raison de 10 à 20 % V/V,

et, le cas échéant, des stabilisateurs jusqu'à concurrence de 1000 ppm.

**2.** Formulation selon la revendication 1,possédant la composition indiquée ci-après :
1 % V/V d'ivermectine, de 65 à 90 % V/V d'huile de sésame, de 10 à 20 % v/v d'oléate d'éthyle et de 1 à 3 % V/V d'alcool benzylique ou 10 % V/V d'huile de ricin, ainsi que, le cas échéant, des stabilisateurs jusqu'à concurrence de 500 ppm.

3. Procédé pour la préparation des formulations selon la revendication 1, **caractérisé en ce qu'**on soumet la substance active à une dissolution (naissante) dans de l'huile de ricin ou dans de l'alcool benzylique et on ajoute les autres composants indiqués à la revendication 1, ou bien **en ce qu'**on dissout la substance active dans un mélange constitué par les composants indiqués à la revendication 1.

4. Utilisation d'huile de ricin ou d'alcool benzylique à titre d'agent améliorant le pouvoir de solubilisation dans une formulation selon la revendication 1.